# EUROPEAN PATENT SPECIFICATION

(11) **EP 3 409 212 B1**
(45) Date of publication and mention of the grant of the patent: **12.02.2020**
(21) Application number: 18158667.8
(22) Date of filing: 26.02.2018
(51) Int. Cl.: A61B 17/00, A61B 17/32

(54) **LAPAROSCOPIC MORCELLATING RECEPTACLE**
LAPAROSKOPISCHER BEHÄLTER ZUM MORCELLIEREN
RÉCEPTACLE DE MORCELLEMENT LAPAROSCOPIQUE

(30) Priority: 20.02.2018 US 201815900758
(43) Date of publication of application: 05.12.2018
(73) Proprietor: Polo, Oscar, Portland, OR 97202 (US)
(72) Inventor: Polo, Oscar, Portland, OR 97202 (US)
(74) Representative: Mewburn Ellis LLP

(56) References cited:
- WO-A1-2015/084769
- WO-A1-2016/058086
- US-A- 5 215 521

## Description

### FIELD OF THE DISCLOSURE

The teachings herein relate to exemplary methods and compositions useful in laparoscopic power morcellation. More specifically, the embodiments herein relate to receptacles configured to prevent morcellated particulate tissue, from spreading into nearby areas from the cutting site.

### BACKGROUND

Power morcellators are devices, used in laparoscopic surgery that morcellate, or cut tissue, into smaller pieces to allow for removal through small surgical access sites. Currently laparoscopic power morcellation for the removal of the uterus (hysterectomy) or uterine fibroids (myomectomy) in women is discouraged because, based on an analysis of currently available data, it may pose a risk of inadvertently spreading cancerous tissue, notably uterine sarcomas, beyond the uterus. In trying to prevent the potential spread of cancerous tissue, morcellation receptacles have been designed to contain the specimen that is being cut and to remove the severed pieces as they are being cut. Current receptacles for laparoscopic surgery are used for retrieving specimen. They have an expandable and collapsing collar at the opening that is attached to a hanging bag that opens enough to accommodate the targeted tissue. Unfortunately, these current receptacles do not give the surgeon the room or visibility to perform power morcellation within them without the risk of severing the receptacle. Other receptacles are designed for morcellation with a hand-held scalpel as opposed to power morcellation. These receptacles have the opening of the bag external to the cavity so that the tissue inside the receptacle can be visibly severed and removed through the expanded incision on the abdominal wall. An inflatable bag design was recently approved for use with both a handheld scalpel or power morcellator, but this bag's small opening significantly limits the use of a power morcellator and laparoscope because there is not sufficient room for the surgical cutting device to move and cut within the bag. Furthermore, this bag is inflatable and so severing or puncturing this bag would make it ineffective for its intended use. Preferred receptacles herein are not inflatable. US005215521 discloses a laparoscopy organ retrieval apparatus and procedures are presented for minimum invasion surgery inclusive of laparoscopic nephrectomy, cholecystectomy and other organ dissection, morsellation removal from the abdomen through a keyhole incision. The apparatus and procedures permit the safe and total removal of an cirgan from a body cavity in a morsellated condition through the combination utilization of an entrapment envelope-sheath. WO2015/084769A1 discloses a retrieval device and method for retrieving tissue from a body cavity. The retrieval device includes a pouch having an aperture and a pouch wall defining an interior space. An inflatable rim is coupled to the pouch and facilitates retrieval of the tissue into the interior space of the pouch. The retrieval device further includes a closure device coupled to the pouch wall for enclosing the tissue within the interior space of the pouch. A portion of the pouch wall includes a self-sealing characteristic that allows an instrument to pierce the pouch wall and the pouch wall is resealable after withdrawal of the instrument. WO2016/058086A1 discloses a device, method and kit for morcellation preventing leakage of severed tissues inside a patient's body during morcellation. The device comprises a tube with a first end for inserting into the body; a sealable enclosure, at the first end, for receiving the tissue inside the body when unsealed; a cutter, at the first end, for morcellating the tissue into a severed tissue in the enclosure when sealed; and a channel coupled with the tube for transporting the severed tissue from the enclosure outside the body.

Accordingly, there is a need in the art for receptacles configured to give the medical practitioner (e.g., surgeon) enough room and visibility to perform power morcellation, within them, using the spaced apart laparoscopic ports already in place for the intended surgical procedure and that allows for improved handling and visibility of the specimen and decreases the likelihood of severing the receptacle. The following disclosure describes expanding/collapsing receptacles configured for use with power morcellators that address this need in the art.

### SUMMARY

The invention is defined by claim 1. Preferred embodiments of the invention are defined by the dependent claims.

Preferred embodiments are directed to a morcellation receptacle system having: a collapsible receptacle, having a vertical axis with a lower half section with a distal area, and an upper half section with a proximal end, and a horizontal axis, configured such that when the collapsible receptacle is collapsed on its horizontal axis, it is linearly sized to fit within and through an opening between 14-20 mm in width but not when expanded in a natural open position; said receptacle comprising: a distal area; a plurality of at least three support rods positioned in a circumferential and equidistant manner around the vertical axis, or substantially so, and having proximal ends extending proximally and laterally away from the distal area and configured such that the support rods can move towards each other on the horizontal axis when the receptacle is collapsing and move away from each other on the horizontal axis when the receptacle is opening, a flexible liner that is water resistant, low-friction, tear-resistant, and made of material different from the support rods, having a thickness between .05 - .15 mm, wrapped along the support rods such as to define a bag with a closed end at the distal area; and a cover that is permanently attached to the perimeter of the proximal end of the receptacle that comprises a substantially central opening wherein said opening is configured to be releasably closable such that at least 75% of the opening's area is occluded, and the cover is expandable and collapsible to fit through the 14 to 20 mm opening. An exemplary method of morcellating a targeted piece of tissue in a subject comprising:
a) providing a collapsible receptacle, having a vertical axis with a lower half section with a distal area, and an upper half section with a proximal end, and a horizontal axis, configured such that when the collapsible receptacle is collapsed on its horizontal axis, it is linearly sized to fit within and through an opening between 14-20 mm in width but not when expanded in a natural open position; said receptacle comprising: a distal area; a plurality of at least three support rods positioned in a circumferential and equidistant manner around the vertical axis, or substantially so, and having proximal ends extending proximally and laterally away from the distal area and configured such that the support rods can move towards each other on the horizontal axis when the receptacle is collapsing and move away from each other on the horizontal axis when the receptacle is opening, a flexible liner that is water resistant, low-friction, tear-resistant, and made of material different from the support rods, having a thickness between .05 - .15 mm, wrapped along the support rods such as to define a bag with a closed end at the distal area; and a cover that is permanently attached to the perimeter of the proximal end of the receptacle, is expandable and collapsible to fit through the 14 to 20 mm opening, and comprises a substantially central opening; b) creating one or more incisions in the subject near the targeted piece of tissue, wherein the incision has a width of between 14-20 mm and inserting a first trocar sleeve into a first incision; c) collapsing the receptacle;
d) inserting the collapsed receptacle inside of the subject through the first trocar sleeve; e) positioning the targeted tissue into the receptacle through the central opening in the cover; f) closing the central opening such that at least 75% of the opening's area is occluded; g) positioning a morcellator into the receptacle and cutting the target tissue; h) removing the cut targeted tissue from the receptacle through a trocar sleeve; and i) collapsing the receptacle and withdrawing it from inside the subject through a trocar sleeve.

### BRIEF DESCRIPTION OF THE DRAWINGS

It will be appreciated that the drawings are not necessarily to scale, with emphasis instead being placed on illustrating the various aspects and features embodiments of the disclosure, in which:
FIG. 1 is a perspective view of a collapsed receptacle passing through a trocar sleeve penetrating a patient's abdominal wall.
FIG. 2 is a perspective view of an opened receptacle having straight support rods coupled to filaments.
FIG. 3 is a perspective view of an opened receptacle having straight support rods not coupled to filaments.
FIG. 4A is a perspective view of an opened receptacle having a drawstring and concave support rods coupled to filaments.
FIG. 4B is a perspective view of an opened receptacle having a drawstring and concave support rods not coupled to filaments.
FIG. 4C shows a perspective view of an opened receptacle having drawstring, preferably made of nitinol wire.
FIG. 5 is a perspective view of an alternative collapsed receptacle passing through a trocar sleeve and having rods that couple to the distal base.
FIG. 6 is a perspective view of an opened receptacle of FIG. 5.
FIG. 7 is a perspective view of an opened receptacle having 2 rods that bend through the distal base.
FIG. 8 is a perspective view of the rods in the receptacle shown in FIGs. 5 and 6.
FIG. 9 is a perspective view of elastic rods that bend through the distal base of the receptacle shown in FIG. 7.
FIG. 10 is a perspective view of a tissue sample being morcellated and removed within an opened receptacle.

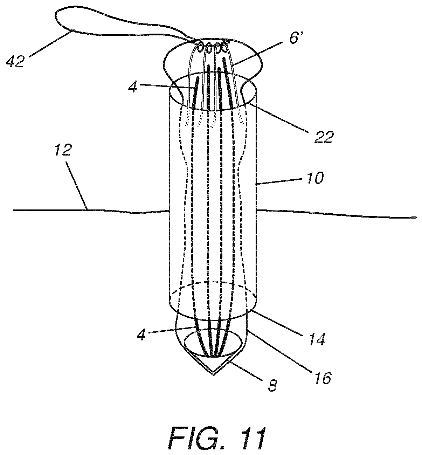

FIG. 11 is a perspective view of a collapsed receptacle having a draw string and positioned within a laparoscopic sleeve penetrating a patient's abdominal wall.
FIG. 12 is a perspective view of an open receptacle having a membrane cover penetrated by four laparoscopic sleeves.
FIG. 13 is a perspective view of an open receptacle having a lid with premade holes configured to receive laparoscopic sleeves and the filaments.
FIG. 14 shows a top view of a lid with four holes spaced equidistantly along the perimeter of the lid (e.g., 90 degrees apart), in a square or diamond configuration.
FIG. 15 shows a top view of a lid with four holes spaced apart. The three filaments and their respective holes form a triangle, that is preferably equilateral such that the filaments are equidistant apart from each other with respect to the triangular formation as opposed to their position on the perimeter of the lid.
FIG. 16 shows a top view of an alternative lid.
FIG. 17 shows a top view of another alternative lid.
FIG. 18 shows a side view of a hook and loop fastener on a lid.
FIG. 19 shows a top view of another alternative lid.
FIG. 20 shows a side view of a hook and loop fastener on a lid.
FIG. 21 shows a top view of another alternative lid.
FIG. 22 shows a valve for releasing air within the receptacle.
Figure 23 shows a perspective top view of another alternative lid.
Figure 24 shows a top view of another alternative lid.
FIG. 25 shows a top perspective view of a specimen being inserted through a closable central opening within a lid.
FIG. 26 shows a perspective view of a receptacle being pulled proximally out of the patient through a trocar sleeve.
FIG. 27 shows a perspective view of an alternate receptacle being pulled proximally out of the patient through a trocar sleeve.
FIG. 28 shows an alternative hook and loop mechanism of closing a central slit on the lids.
FIG. 29 shows a top view of a zippered lid in a closed configuration.
FIG. 30 shows a top view of a zippered lid as it is partially open.
FIG 31 shows a perspective view of a receptacle with a zippered lid being pulled proximally out of the patient through a trocar sleeve.

### DETAILED DESCRIPTION OF THE ILLUSTRATED EMBODIMENTS

Embodiments of the present disclosure are described below. It is, however, expressly noted that the present disclosure is not limited to these embodiments, but rather the intention is that modifications that are apparent to the person skilled in the art and equivalents thereof are also included.

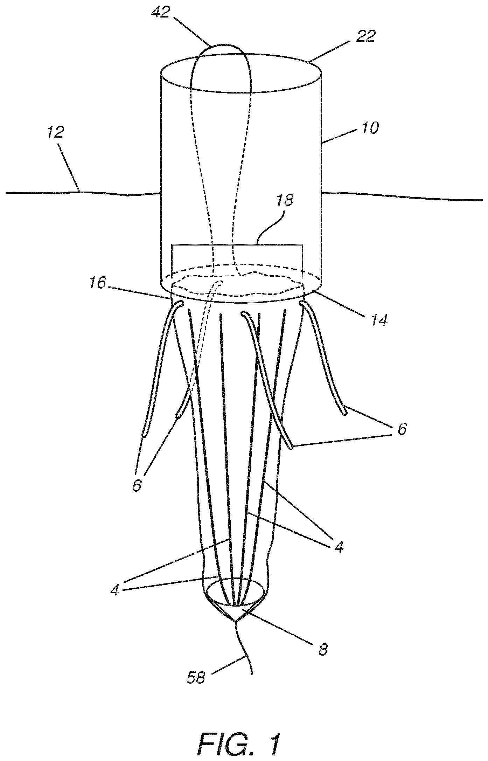

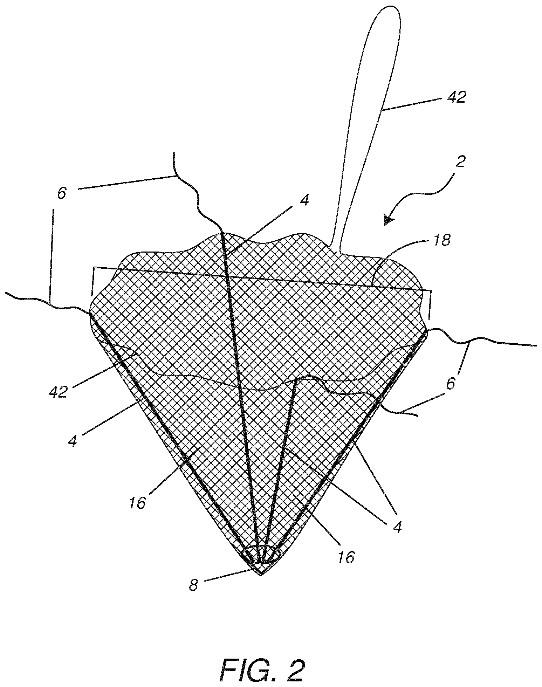

FIGS. 1 and 2 show the receptacle 2 in a collapsed/closed position and an open position respectively. The collapsed configuration shown in FIG. 1 and the open configuration of FIG. 2 both depict the receptacle 2, having a plurality of support rods 4 coupled to a distal base 8. The receptacle 2 has a vertical axis with a lower half section and a distal end, and an upper half section with a proximal opening 18.

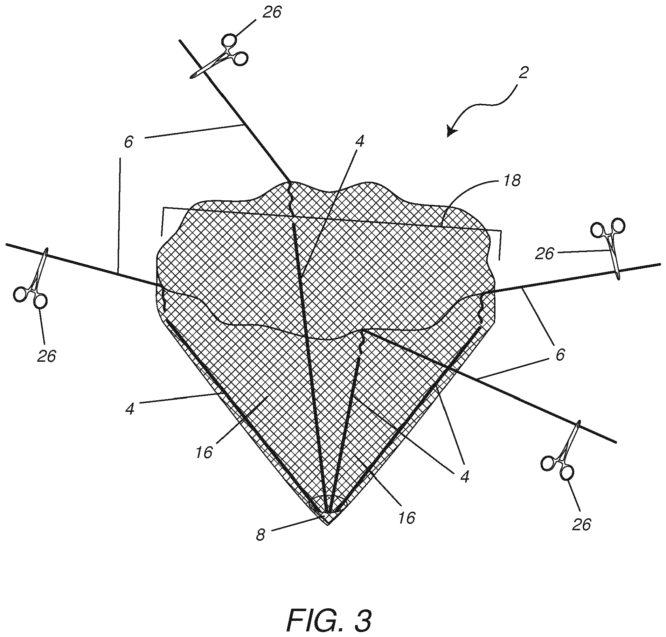

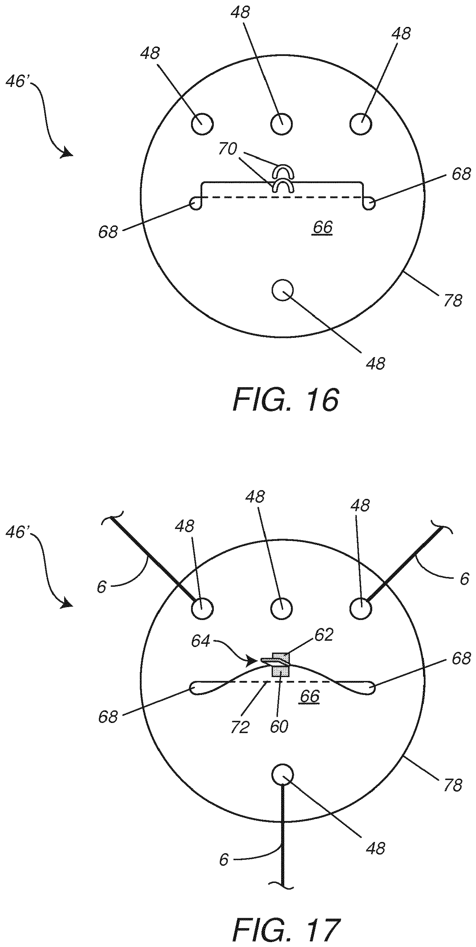

In FIG. 1, the closed receptacle 2 is shown passing through the inside of a trocar sleeve 10, which can non-exclusively include any suitable laparoscopic port or sleeve used in surgery. The receptacle has a liner 16, made of a thin, flexible material that is fluid impermeable, and preferably is hypoallergenic, has a low coefficient friction, and is made of a tear-resistant material such as ripstop nylon, polyurethane, or polyisoprene. Latex could be a suitable material as well, such as for situations where neither the patient nor medical practitioner is allergic. The flexible liner 16 is wrapped along the support rods 4 (it can be on the outside and/or inside of the rods 4) such as to define an enclosed space, or bag, with a proximal opening 18 defined by proximal/upper portion of the flexible liner 16. According to preferred embodiments, such as when the rods 4 are flexible, they can maintain the shape of the open receptacle 2, such as a bowl shaped, for example. According to further embodiments, the rods are coupled to the bag in a direction which keeps the rods from sliding right or left. In FIG. 1, the proximal ends of the support rods 4 traverse up from the distal base 8 to the proximal opening 18. This is merely optional, however. As an alternative, the support rods 4 only traverse up to the upper proximal section of the flexible liner 16, such as shown in FIG. 3. Advantageously the flexible liner 16 creates a waterproof seal, water resistant, or impermeable barrier to blood or particular tissue around the working space, with the exception of the proximal opening 18. Thus, according to certain embodiments, the proximal opening 18 is the only opening, or access point, of the receptacle 2. According to further embodiments, such as shown in FIGs. 16-21, a covering (e.g., lid 46') can be placed over the proximal opening 18 wherein the covering has a central opening 68 configured to allow a specimen to pass through, and that can be covered, or substantially so.

The trocar sleeve 10 is positioned through a patient's outer body, or more specifically their abdominal wall 12 such that its proximal opening 22 is located outside of the patient which leads through the hollow trocar sleeve 10 to a distal opening 14 within the patient's body, or more specifically their abdominal cavity. According to preferred embodiments, in the collapsed configuration, the receptacle 2 has a small enough width or diameter to fit within the trocar sleeve 10 and pass through the proximal and distal openings 22 and 14. Preferred embodiments of receptacles 2 can fit through a trocar sleeve 10 having a hollow internal channel with a diameter or width of about 15 mm, such as 10-20 mm. These dimensions are also applicable for the proximal and distal openings 22 and 14 of the trocar sleeve 10. This configuration can be accomplished using any suitable dimensions, for example, by measuring or approximating the width of the collapsed rods 4, so that at maximal measurement or approximation of the diameter of collapsed rods 4 is less than the diameter/width of the hollow channel within the trocar sleeve 10. Suitable diameters/widths of the collapsed rod can thus be about 14 or 14.5 mm including between 9-19 mm. Similarly, the distal base 8 should be sized to likewise fit though the hollow channel within the trocar 10 and its proximal and distal openings 22 and 14. Thus, in general, the distal base's width/diameter should be less than the diameter/width of the hollow channel within the trocar sleeve 10, including the proximal and distal openings 22 and 14. These widths/diameters can be about 14 or 14.5mm including between 9-19 mm. Embodiments herein further contemplate instances where the rods 4 and/or distal base 8 are made of flexible or elastic material, and their widths/diameters in a closed configuration could be the same or larger than the width/diameter of the hollow channel within the trocar sleeve 10, including the proximal and distal openings 22 and 14, so long as the rods 4 and distal base 8 can further compress/collapse to pass through the trocar sleeve. As a non-exclusive example, the support rods 4 and/or the distal base 8 can be made of an elastic material such as nitinol, also known as nickel titanium.

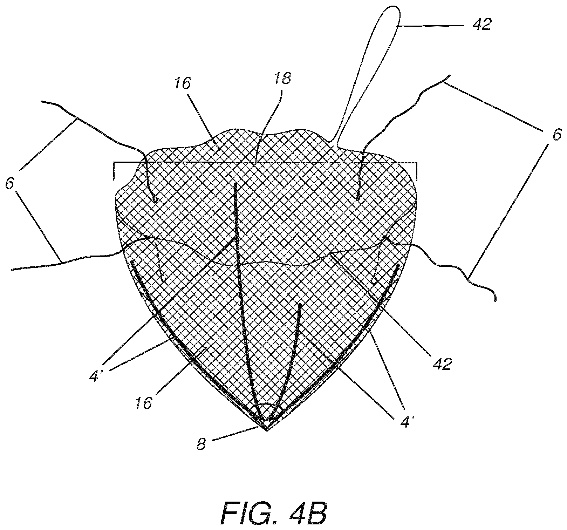

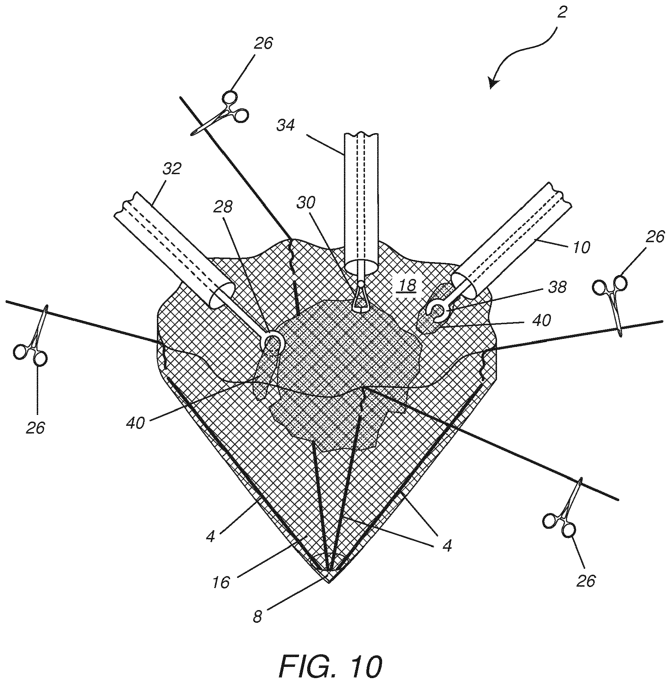

As shown in FIG. 2, the receptacle 2, in its open position, is shaped as a cone or bowl, for containment of the specimen, trocar sleeves, and surgical instruments, such as graspers, laparoscopes, and the morcellator. While non-limiting, it is preferred that the receptacle 2 is in a bowl shape rather than a funnel shape to accommodate the specimen 40 better. The receptacle 2 can also be cone shaped or concave as well. A main objective of the present embodiments is to provide a receptacle 2 that is small enough to travel through a trocar sleeve 10 (whether through insertion or extraction), yet also expand large enough to surround a working space which can include a target specimen 40 and multiple trocar sleeves and instruments for cutting, grasping, viewing, and extracting the specimen from the patient. FIG. 10 is an exemplary figure showing various instruments 28, 30, 38 grasping and cutting a specimen 40 that the receptacles 2 herein are configured to surround when positioned inside a patient. Accordingly, preferred proximal opening 18 diameters are between 14-30 cm, when the receptacle 2 is in an open position, such as shown in FIGs. 2 and 10. The proximal opening 18 can be of any suitable shape to accommodate the specimen 40 and desired instruments, such as circular or oval, for example. The proximal half of the cone shaped bag can include a collar of 2-5 cm in length that is made of the same fabric that is continuous with the vertical axis of the bag and maintains the shape throughout its length. In FIG. 4B, the area of liner 16 between the proximal ends of the rods 4 and the proximal opening 16 can be considered the collar.

In FIG. 2, the receptacle 2 has a distal base 8 that is operably coupled to a plurality of support rods 4 which extend to proximal ends that define a proximal opening 18 of the receptacle 2. In other embodiments, the support rods 4 and 4' do not extend to the proximal opening 18, such as shown in FIGs. 3 and 4B. In FIGs. 3 and 4B, the support rods 4 and 4' extend proximally to the upper half of the receptacle 2, but not to the perimeter of the proximal opening 18. While discussion herein is primarily directed to support rods 4, those with skill in the art will appreciate that disclosure herein pertaining to these features are readily interchangeable to other shapes of support rods, such as support rods 4', where suitable. The distal base is also couple to the distal end of the liner.

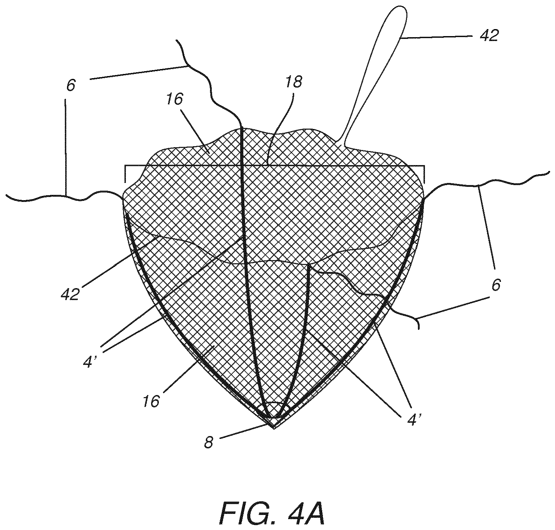

The proximal ends of the support rods 4 and are configured to move towards each other when the receptacle 2 is collapsing and move away from each other when the receptacle 2 is opening. According to preferred embodiments, the receptacle 2 has a frame of (3-8) of axial support rods 4 that are circumferentially, attached to the distal base 8, preferably at equidistant lengths from each other. These numbers are non-limiting, but preferably four rods 4 are used. The support rods 4 can be shaped in any suitable manner, non-exclusively including straight rods, or curvilinear, such as concave and convex rods, and other suitable shapes, when in the expanded position. FIG. 2 shows an open receptacle 2 having straight support rods 4 that distally converge from their proximal ends to the distal base 8. FIGs. 4A and 4B show an open receptacle 2 having concave support rods 4' that distally converge from their proximal ends to the distal base 8. Additionally, the support rods 4 can non-exclusively be: rigid, adjustable, hinged, jointed, telescoping, flexible, or elastic. Preferably the rods 4 have a natural spring tension that allows them to expand outward when not confined within the trocar sleeve 10 and to collapse inward when traveling through the hollow channel of the trocar sleeve 10.

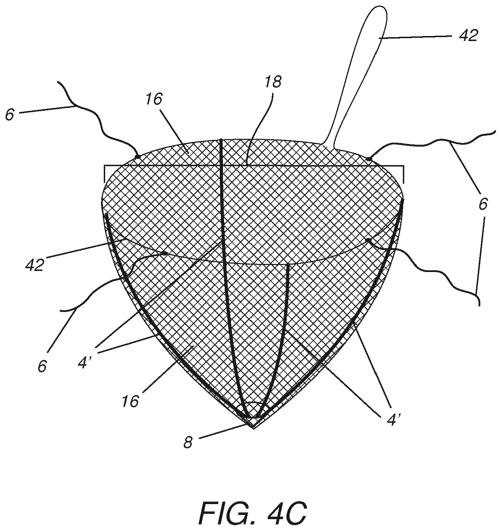

FIGs. 1 and 2 also show a drawstring 42 that circumnavigates around the vertical axis of the proximal opening 18 of the receptacle and configured such that when the drawstring 42 is tightened, the opening of the receptacle narrows; the proximal ends of the support rods move toward each other, thereby collapsing the receptacle on its horizontal axis. The drawstring 42 can be coupled to the flexible liner 16 using any suitable way, such as threading the drawstring 42 through loops or a channel within the liner 16 or weaved/threaded through the liner 16 itself. The drawstring 42 can be tightened using any suitable method, such as pulling it in a proximal direction whether manually or with an instrument. Conversely, when the drawstring 42 is loosened, the proximal ends of the support rods move away from each other, thereby expanding the bag to its open position, such as shown in FIG. 2. The drawstring 42 can be made of any suitable material that either has elasticity or does not and will aid in expansion of the proximal end and help maintain the liner 16 on the proximal end from folding on itself when in the open position such as nylon, or thin nitinol, such as .25 mm caliber nitinol. FIG. 4C shows a drawstring 42 that is made of nitinol and wherein the filaments 6 are not attached to the rods 4' but the perimeter of the proximal opening 18. According to further embodiments, the drawstring 42 is not elastic, and the rods and filaments are configured to keep the proximal end expanded.

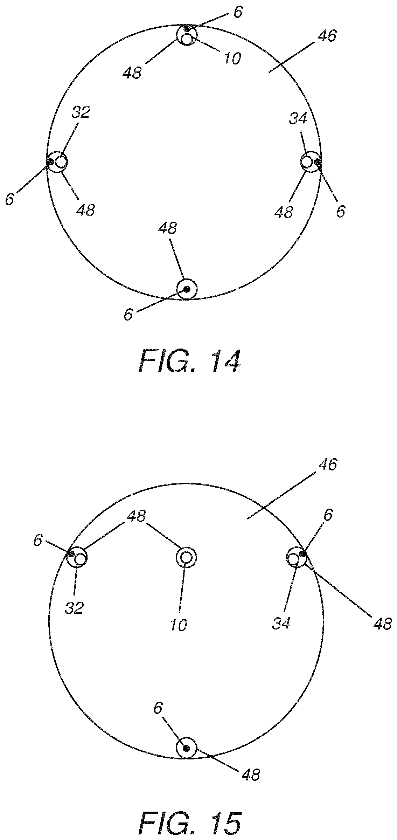

The receptacles herein have a plurality of at least three, or four, support filaments 6 having distal ends coupled to the upper half section of the receptacle in a circumferential manner and configured such that when proximal ends of the support filaments are pulled away from each other, the receptacle 2 is moved in a proximal direction and is maintained in an open position. According to preferred embodiments, the proximal ends/sections of the filaments 6 are brought out of the anterior abdominal wall and can be anchored using any suitable device such as pins or clamps 26 to stabilize the receptacle 2 and maintain it in its open position, such as shown in FIG. 10. The filaments 6 can be spaced apart equidistant around the receptacle 2, (e.g., 90 degrees apart from each other) see FIG. 14 square configuration, or otherwise sufficiently apart from each other, such as at least 45 degrees from each other around the circumference of the receptacle 2. The filaments 6 can be a string or made of any suitable material with elasticity that are configured to stand erect in their natural position for easier grasping by the surgeon. Preferred filaments 6 are connected to the proximal end 18 of the receptacle 2 and not weaved through the collar, to prevent the receptacle 2 from folding in on itself. Preferred filaments 6 are made of thin nitinol, including .25 mm caliber nitinol.

According to certain embodiments, the proximal sections of the rods 4 can be coupled to filaments 6 (see FIGs. 2 and 4A) such that when pulled close inwardly, the rods 4 collapse so that the receptacle 2 can pass through the trocar 10 whether being inserted or extracted. Likewise, by pulling the proximal end of the rods 4, such as through pulling the filaments 6 outwardly, the proximal sections of the rods 4 diverge during opening. The filaments 6 can be coupled to any part of the upper half section of the receptacle 2, including the proximal sections of the rods 4, the flexible liner 16, or the rim around the proximal opening 18 of the receptacle, using any suitable way, including adhesives, tying, heat, etc.

For purposes of this description there are preferably two to four filaments 6, but more support filaments can be attached and anchored anteriorly, to stabilize the receptacle 2 during morcellation. Even more preferably, the embodiments herein use 3 or 4 filaments 6 spaced apart from each other equidistantly. Additionally, FIG. 14 shows four filaments 6 spaced every 90 degrees around a circular lid 46. FIG. 15 shows three filaments that aren't arranged equidistantly apart from each other around the perimeter of the lid 46, but form a triangle, which could be an equilateral triangle. For both FIGs. 14 and 15 the trocar 10 is preferably inserted through the navel of the patient.

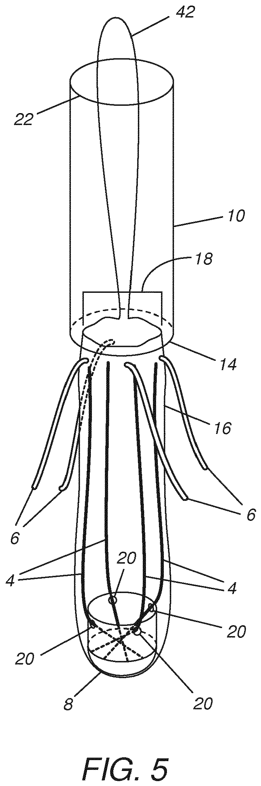

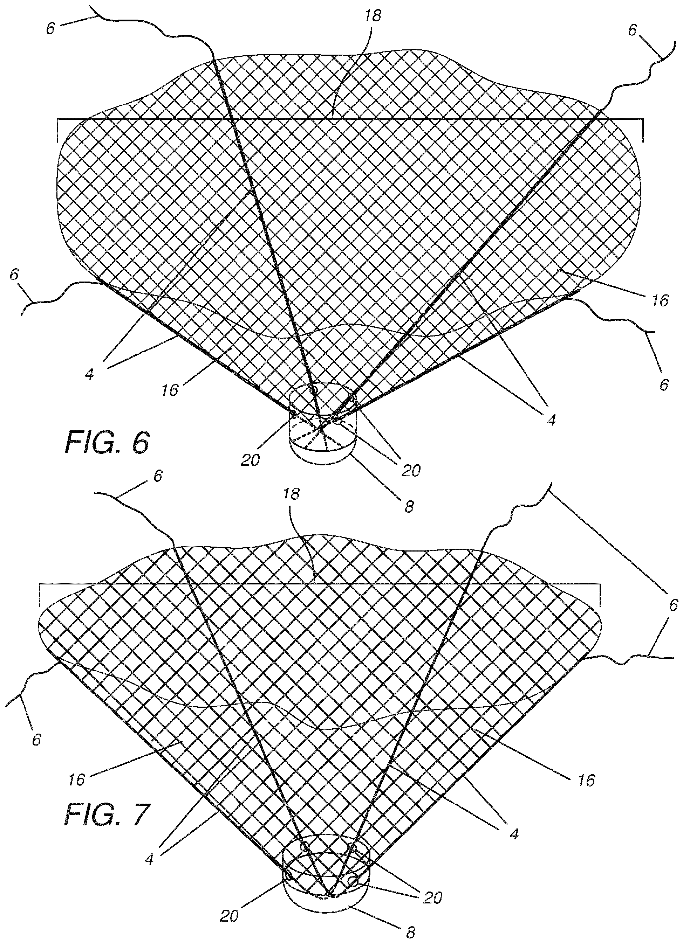

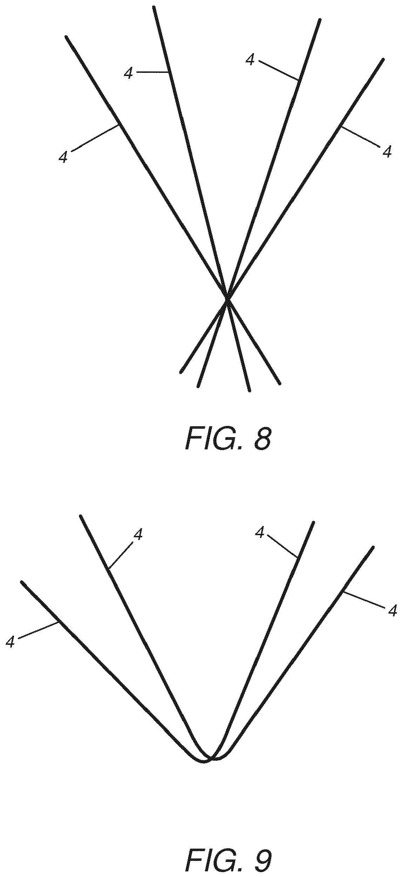

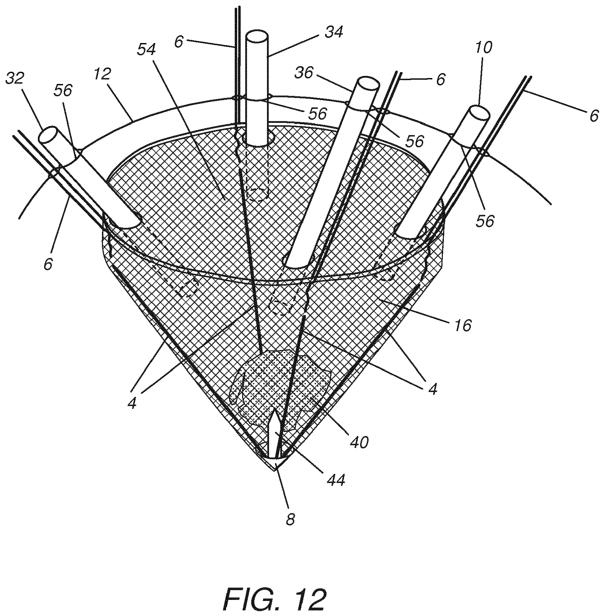

The distal sections of the rods 4 can be coupled to the distal base 8. While the rods 4 described herein can couple to the distal base 8 in any suitable way, FIGs. 5 and 6 depicts a half-capsule shape (half of a spherocylinder) distal base 8 having apertures 20 configured for receiving the distal sections of the rods 4. FIG. 8 is a close-up view of the distal sections of the four rods 4 that couple to the distal base 8 shown in FIGs. 5 and 6. In contrast, FIG. 7 also depicts a half-capsule shape (half of a spherocylinder) distal base 8 having apertures 20 configured for receiving the distal sections of the rods 4. According to this embodiment, the rods 4 curve through the distal base 8, thereby providing natural spring tension when the rods 4 are compressed within the trocar 10 so they can spring open upon passing through the trocar's distal opening 14. FIG. 9 is a close-up view of the distal sections of elastic rods 4 that do not connect to the distal base 8, but rather form a U or V-shape. FIG. 10 shows a targeted tissue specimen 40 and multiple instruments grasping and cutting it within an open receptacle 2. The tissue grasper 38 can serve two purposes: one to help stabilize the tissue 40 as it is being cut and the other is to pull the cut pieces out of the patient, such as through the main trocar sleeve 10, which can be any suitable size, but is preferably 15 mm, or 10-20 mm. The power morcellator 28 is shown cutting the tissue specimen 40 and is positioned through a trocar sleeve 32 that is preferably about 8 mm (e.g., 5-8 mm) in diameter, but can be any suitable size. A second tissue grasper 30 traversing through its trocar sleeve 34 is shown that can optionally help stabilize the tissue specimen 40 during morcellation. This trocar sleeve 34 is preferably about 8 mm in diameter but can be any suitable size. Additional instruments can likewise be utilized within the open receptacle 2, such as a laparoscope. FIG. 12 shows a fourth trocar sleeve 36 that a laparoscope or other instrument can be traversed though. The fourth trocar sleeve 36 can be any suitable diameter such as 5 mm, for example.

In FIG. 12, the proximal opening 18 of the receptacle 2 is covered by a membrane lid 54, that is collapsible when the receptacle 2 is in a closed position, and can expand, or flatten out, when the receptacle 2 opens. The membrane 54 is thin enough that trocar sleeves 10, 32, 34, and 36 can be pressed down manually and puncture the membrane at holes 56, such that the instruments 28, 30, and 38 can be positioned into the open receptacle 2. Any suitable number of trocar sleeves can puncture the membrane 54 depending on the circumstances. For example, 2-6 trocar sleeves could be used to puncture the membrane 54. FIG. 12 also shows a spike 44 that extends proximally from and is operably coupled to the distal base 8. According to these embodiments, the user can pierce the tissue specimen 40 onto the spike 44 thereby helping to stabilize the tissue 40 during cutting.

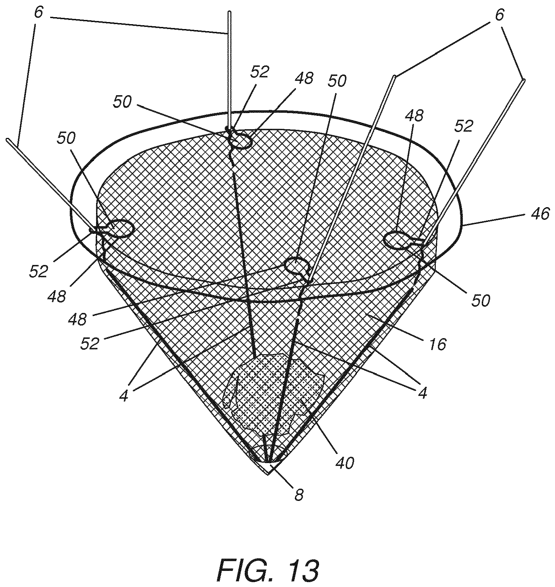

FIG. 13 shows a lid 46 that covers the proximal opening 18 of the receptacle 2 and is collapsible and can expand (flattens out) when the receptacle 2 opens. The lid 46 can be identical to the membrane lid 54 with the exception that it has premade holes 48, and is interchangeable with the embodiments herein, where suitable. The lid 46 is thin and can either be separate/removable from the receptacle 2 or permanently attached to the receptacle 2. Preferably, the lid is sized to fit through an opening of between 14 to 20 mm when collapsed, but not when expanded in its natural open position. The lid 2 can comprise an elastic ring made of .6 mm nitinol and is covered by a thin elastic material such as 1 to 3 mil gauge polyurethane film. The lid 46 includes premade holes 48, in contrast to the penetrable membrane 54. The openings 48 in FIG. 13 can be cut in any suitable shape but are preferably configured to receive at least the trocar sleeves 10, 32, 34, and 36 and preferably also the filaments 6. The number and size of the openings 48 can readily be modified depending on the number of trocar sleeves or instruments desired in the working space within the receptacle 2. This number can be 2-6, for example. Preferred openings 48 in the lid 46 are configured to receive both trocar sleeves and the filaments 6. As shown in FIG. 13 the openings 48 are shaped made in a keyhole shape, having both large 50 and smaller 52 sections. The trocar sleeves, e.g., 10, 32, 34, and 36 can pass through the larger holes 50 while the filaments 6 can pass through the smaller sections 52. The openings 48 can be keyhole shape or other shapes such as slits. Additionally, any suitable number of openings 48 can be used to match and accommodate the number of trocar sleeves and/or filaments. According to preferred embodiments, the size of the openings 48 are sized to receive their designated trocar sleeve. According to preferred embodiments the filaments 6 can be configured to align the trocar sleeves with their designated openings 48.

FIG. 16 shows a non-removable, non-hinged, or permanent lid **46'** covering the receptacle with premade holes **48** and an outer ring which can be a perimeter **78,** and can be made from ripstop nylon, for example. Under this embodiment, the lid is an upper covering, permanently attached to the perimeter of the receptacle **2** at its proximal end that is not configured to be opened, detached from its perimeter **78** (or alternatively a drawstring **42**) or otherwise removed. While they both surround the lid or cover, the perimeter **78** is different from the drawstring **42** in that it cannot be tightened or loosened. In contrast, the central portion of the drawstring **42** is configured to tighten (shrink in diameter) when pulled outward and loosen (enlarge in diameter) when pulled inward. Preferably the drawstring **42** is made of a pliable material in addition having some elasticity. Under certain embodiments, pulling oppositely on the drawstring **42** and the distal cable **58** can collapse the receptacle.

According to certain embodiments the lid 46' has a central, or substantially central opening or aperture 68 that can be closed and opened. The central opening preferably has a center that is concentric with the center of the lid. The central opening **68** can be configured to be entirely closed or substantially so, such that at least 75% of the opening is closed. More specifically, the edges (**72**) can be approximated so that they are adjacent to or overlapping each other resulting in over 75% occlusion or closure of the central opening **68.**

Likewise, the opening can be configured to be entirely open or substantially so, such that at least 75% of the opening is open. According to preferred embodiments, the central opening **68** is sized to allow a large specimen to be contained within the receptacle **2** and is thus larger than the premade holes **48,** which are preferably configured to be used with trocars. The central opening can preferably be between 10 - 30 cms in length, for example. All lids described herein can be made of any suitable pliable material such as nylon (e.g., 7 denier ripstop), TYVEK®, or Ultra-High-Molecular-Weight Polyethylene, such as DYNEEMA®.

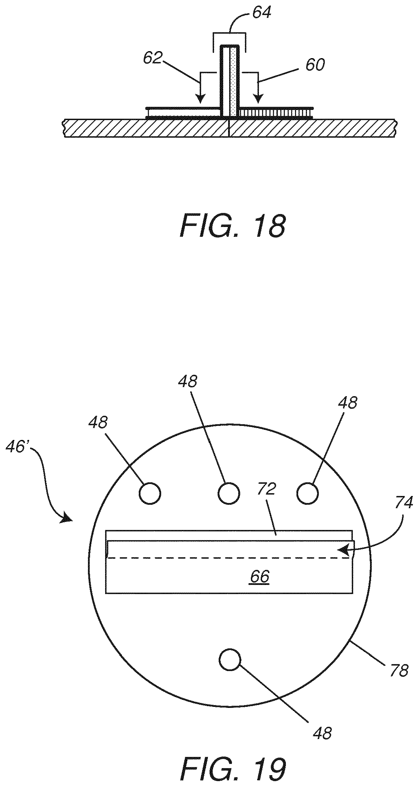

As shown in FIG. 16, a flap **66** on the lid **46'** can be configured to substantially or completely cover the central opening **68.** The flap **66** can be releasably connected to the other side of the central opening **68** by any suitable means, including adhesives, buttons, snaps, or hook and loop fasteners, for example. In FIG. 16, the flap **66** can be secured by an adhesive, such as a wetness activated sealant, to the other side of the central opening **68.** The wetness activated sealant can be made of a hydrophilic material such that when wetted (e.g., by saline), the cohesive bond can be formed with the other side of the central opening **68.** FIG. 19 also shows the use of an adhesive or sealant. In this embodiment, the underside of the flap **66** includes a sealant/adhesive that binds with the topside of an under flap **72.** The section of overlap **74** can be bound by the resulting cohesive bond. The over flap **66** and the under flap **72** can also be coupled by other mechanical, or non-adhesive ways, such as hook and loop fasteners, for example.

The flap **66** can also include a proximal protrusion/proximal extension member that extends proximally away from the lid **46'** surface. For example, in FIG. 16 one or more loops **70** protrude proximally from the flap **66** or lid **46'** and can be used by the practitioner to grasp and pull proximally, such as to extract the receptacle **2** from the patient. The loops **70** (and other suitable proximal extension members described herein) have an additional utility in that a practitioner can grasp one or more of them to keep the central opening **68** open, such as when they are placing the specimen **40** into the receptacle **2.** Additionally, and as shown in Figs. 17 and 18, hook **60** and loop **62** fasteners can be used to cover the central opening **68** to create a proximal protrusion tab **64.** As one example, the loop **62** section can be positioned on the flap **66** and the hook **60** section can be positioned on the opposing side **72** of the central opening **68.** These positions can be interchanged. The hook **60** and loop **62** fasteners can be used like the loops **70** described above. Specifically, they can be grasped and pulled proximally, such as to extract the receptacle **2** from the patient and the practitioner can grasp one or more (**60** and **62**) fasteners to keep the central opening **68** open, such as when they are placing the specimen **40** into the receptacle **2.** FIG. **18** shows a slightly different embodiment than Fig. 17 in that two sides of the lid **46'** are brought together by the hook **60** and loop **62** fasteners to close the central opening **68,** instead of a flap **66** overlapping the central opening **68.**

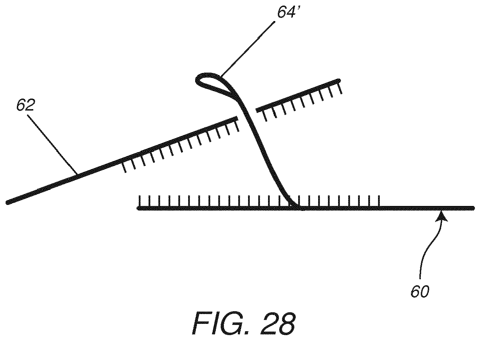

Additionally, FIG. 28 shows another way of closing the central opening **68** by using hook **60** and loop **62** fasteners. In this embodiment, a proximal protrusion tab **64'** extends upward from the hook **60** section through an aperture in the loop **62** section, these sections can be interchanged as well. In this embodiment, pulling up on the proximal protrusion tab **64'** can close the hook **60** and loop **62** fasteners to each other. Further pulling on the proximal protrusion tab **64'** can actuate the collapse of the receptacle **2** whether in conjunction with the simultaneous distal pulling of the distal cable **58** or by itself using an instrument through a trocar sleeve, for example.

These proximal protrusions/proximal extension members (e.g., loops **70, 84** and tabs **64**) can be configured such that when pulled proximally they actuate the collapse of the receptacle **2** for passing through the trocar sleeve **10.** Preferably these protrusions are centrally located on a non-detachable lid with an outer perimeter **78,** (without a drawstring **42**) and configured such that the simultaneous pulling of the distal cable **58** with the proximal pulling of these protrusions collapses the receptacle **2** so that it can pass through the trocar sleeve **10.** The proximal protrusions can be made of either a rigid or pliable material. A further option, is to incorporate a distal cable **58** that can work in conjunction with the proximal protrusions to collapse the receptacle **2** when pulled distally simultaneously. This configuration can be used in lieu of a drawstring **42** for collapsing of the receptacle **2** for example. It is also envisioned to use a non-detachable lid **46'** having a central opening **68** but lacking the premade holes **48** and instead having a penetrable material as in the membrane embodiments discussed herein. Further preferred embodiments are directed to lids **46'** having premade holes **48** that are covered with a penetrable material and can be transparent. Preferred materials for the penetrable material include polyurethane, for example. Polyurethane film is advantageous in that it has some elasticity and so it can cling to the grasper (e.g., **28** and **38**) that is passing through the hole **48.** Preferred graspers can be between 5-10 mm in diameter, for example. Additionally, the premade holes **48** can be slits that are relatively closed with a thin opening that are widened/expanded when a trocar sleeve (e.g., **32**) is inserted within. This embodiment helps prevent cut material from exiting the receptacle **2** through these oversized holes **48.**

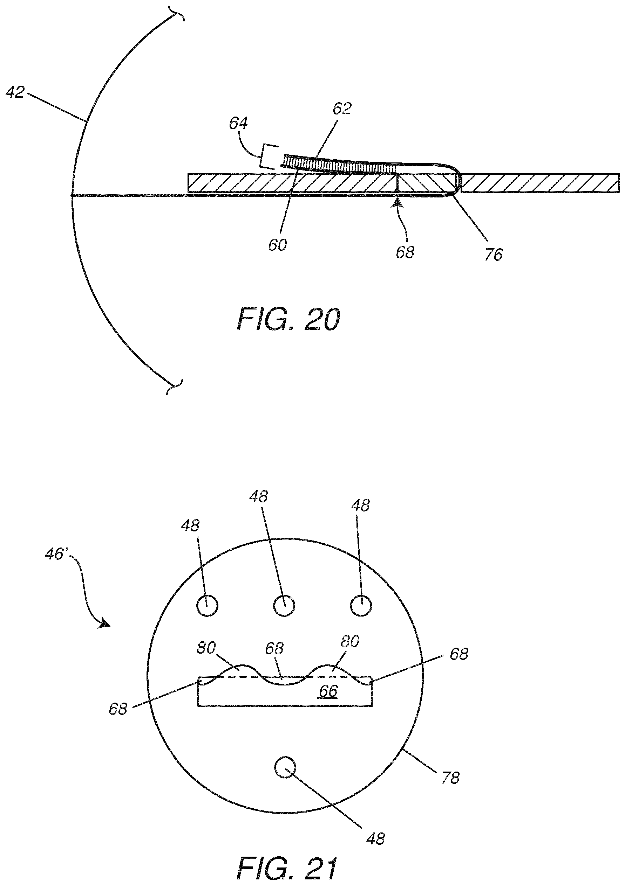

FIG. 21 shows another lid **46'** embodiment wherein the flap **66** is configured to intermittently overlap over the central opening **68.** Under this embodiment, one or more sections **80** of the flap **66** extend to the other side of the central opening **68** and can be coupled accordingly to the embodiments described herein. According to more specific embodiments, the intermittent sections **80** can represent a sinusoidal wave exposing more areas of the central opening **68.** Other flap **66** shapes can include a tongue shape, or straight, or other suitable configurations. The underside of the flap **66** can also include traction gripping members, such as checker plated, studded, or corrugated to increase friction and help maintain the closure over the central opening **68.**

FIG. 20 shows a more intricate coupling mechanism for covering the central opening **68.** According to this embodiment, a loop **62** section extends over the central opening **68** and couples to a hook **60** section on the other side to create a proximal tab **64.** Preferably a thread **76** or other pliable elongated material is attached to the underside of the loop **62** section and traverses under the lid **46'.** This configuration can be reversed such that the thread **76** is attached to the hook **60** section as well. This thread **76** or pliable material can be coupled to the draw string **42** of the lid **46'.** According to certain embodiment, the draw string **42** can be rigid, elastic, or pliable, and can be made of nitinol according to further embodiments.

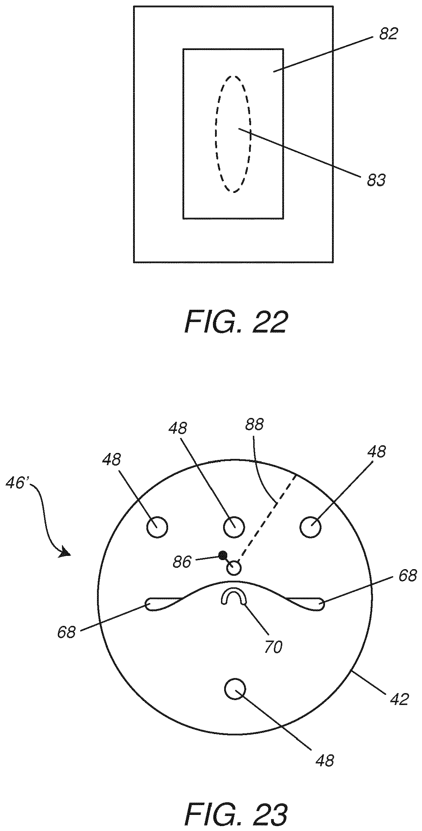

FIG. 23 shows a flap **66** covering most of the central opening **68** and having one or more loops **70** allowing a practitioner to more easily grasp, such as to open the central opening **68** to insert the specimen **40** and to pull proximally to extract the receptacle **2.** This embodiment further utilizes a pull cord tab **86** that extends proximally from the lid **46'** and configured to be grasped by the practitioner. The pull cord tab **86** is coupled to a taught pull cord 88 that is in turn coupled to the drawstring **42.** The configuration is activated when the pull cord tab **86** is pulled, which in turns pulls the taught pull cord **88** towards the center of the lid **46'** which then helps collapse the drawstring **42.** The pull cord **88** can be passed through the one or more loops **70** and then proximally to collapse the receptacle **2.** Collapsing the receptacle **2** is useful when the practitioner desires to extract the receptacle out of the patient.

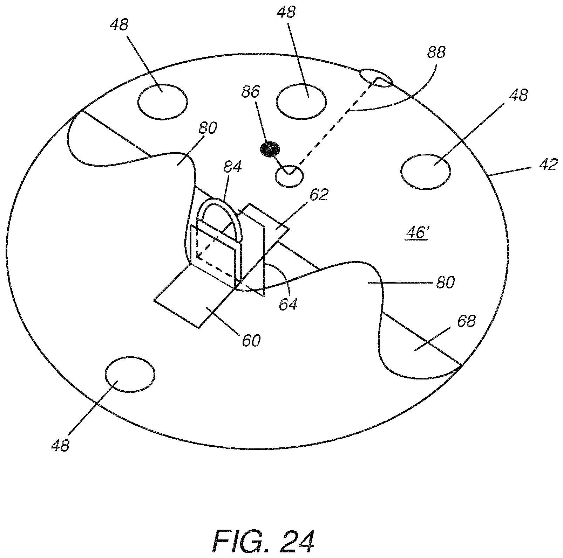

FIG. 24 shows intermittent sections **80** of a flap **66** covering most of the central opening **68** and having a hook and loop fasteners **60** and **62** to couple together to form a proximal protrusion tab **64.** Either the hook and/or loop section **60** and **62** can have a pull loop **84** to make the proximal protrusion tab **64** easier to grasp by an instrument when the practitioner wants to pull the receptacle **2** proximally. This embodiment further utilizes a pull cord tab **86** that extends proximally from the lid **46'** and configured to be grasped by the practitioner. The pull cord tab **86** is coupled to a taught pull cord **88** that is in turn coupled to the drawstring **42.** The configuration is activated when the pull cord tab **86** is pulled, which in turns pulls the taught pull cord **88** towards the center of the lid 46' which then helps collapse the drawstring **42.** The pull cord **88** can be passed through the pull loop **84** and then proximally to collapse the receptacle 2. Collapsing the receptacle **2** is useful when the practitioner desires to extract the receptacle out of the patient.

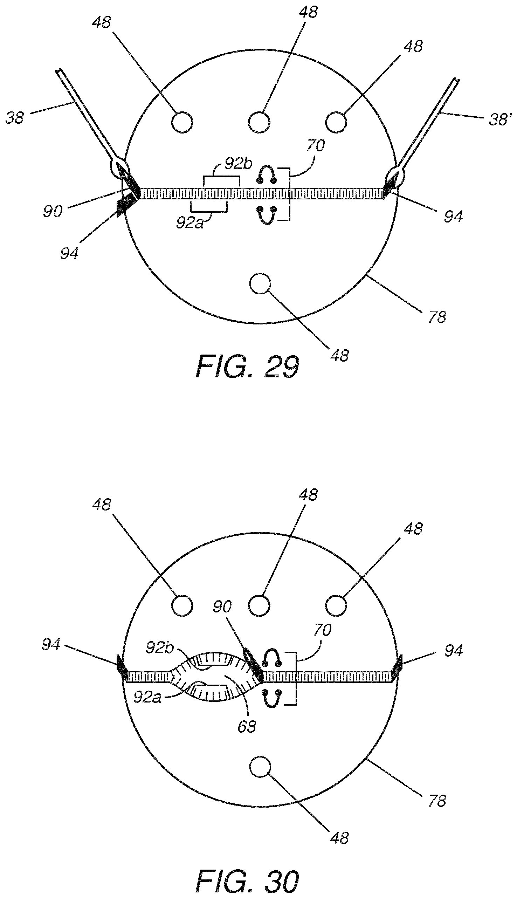

In addition to flaps, a zipper can be used to close or open the central opening **68.** Non-exclusive examples of zippered lids **46'** are shown in FIGs. 29-31. In FIG. 29, a first track of zipper teeth **92a** runs parallel to and are engaged to a complementary second track of zipper teeth **92b** thereby closing the central opening **68.** Under this embodiment, the central opening **68** can be completely closed, such as during morcellation. A zipper slider **90** is operably coupled to both first and second tracks **92a** and **92b** that is configured to open and close the zipper by moving in a first and opposite direction respectively. Preferably the zipper slider **90** has a loop or tab extending from it that can be grabbed by a grasper (e.g., **38**) to slide it more easily or to grab it to pull proximally to collapse and extract the receptacle **2** from the patient. The zipper slider **90** can be moved in either direction, depending on whether opening or closing, by utilizing any suitable instrument, such as grasping instrument **38** traversing through a trocar sleeve **10.** According to preferred embodiments, in addition to the grasping instrument **38** controlling the slider **90** a second grasping instrument **38'** can be used to pull the zipper away from the direction the slider **90** is moving, to provide resistance and make opening and closing easier. According to certain embodiments, one or more resistance tab/extensions **94** can be provided for the second grasping instrument **38'** to pull against.

FIG. 30 shows the central opening **68** partially unzipped, but can be completely unzipped (e.g., opened) as well, such as when inserting or removing the specimen **40.** Similarly, the central opening **68** can be closed after the specimen **40** is inserted and during morcelation. This helps prevent unwanted cut up bits of the specimen from traveling outside of the receptacle **2.** A stop can be used to prevent the zipper slider **92** from undesirably contacting the lid **46'** material. Preferred zipper teeth, sliders, and stops are made of a polymers or plastics. In addition to complementary tracks of zipper teeth, unless specified otherwise, the term "zipper" as used herein, also encompasses other non-teethed coupling profiles, such as rib and channel/groove mating incorporated in sandwich bags. Slider devices used for mating coupling profiles by moving in one direction and disengaging coupling profiles by moving in the opposite direction, such as used in sandwich bags, can likewise be used with these embodiments.

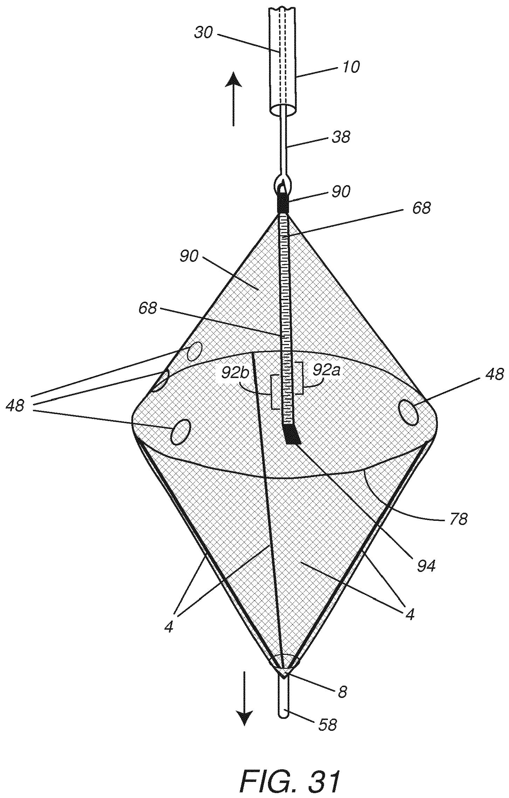

Fig. 31 shows a preferred way of collapsing and removing the receptacle **2** through the trocar sleeve **10.** An instrument **30** extending through the distal opening of a trocar sleeve **10** can grasp any suitable proximal extending member such as the proximal protrusion tab **64,** loop **70,** zipper **90** or pull loop **84** and pull proximally towards the trocar sleeve **10.** Preferably the proximal extending member is centrally located on the lid **46',** or substantially so, to make collapsing of the lid **46'** more uniform. According to further embodiments, the distal cable **58** can be simultaneously pulled distally with another instrument through another trocar sleeve to assist with collapsing the receptacle **2** such that it will fit through the trocar sleeve **10.** According to these embodiments, no drawstring (**42**) is utilized, rather the lid **46'** has an outer perimeter **78.** Additionally, when the one or more proximal extending members (e.g., loops **70**) are pulled proximally, the receptacle is configured to collapse such that its diameter that can fit through the trocar **10** for extraction. According to more specific embodiments, the collapsing of the receptacle **2** during insertion can be facilitated as the expanded sides of the receptacle **2** are pushed into the proximal end of the trocar sleeve **10.** Similarly, the collapsing of the receptacle **2** during extraction can be facilitated as the one or more proximal extending members on the lid **46'** are pulled proximally, and the pulled lid **46'** is pulled into the distal end of the trocar sleeve **10.** This is preferably done while the distal cable **58** is simultaneously pulled in a distal direction.

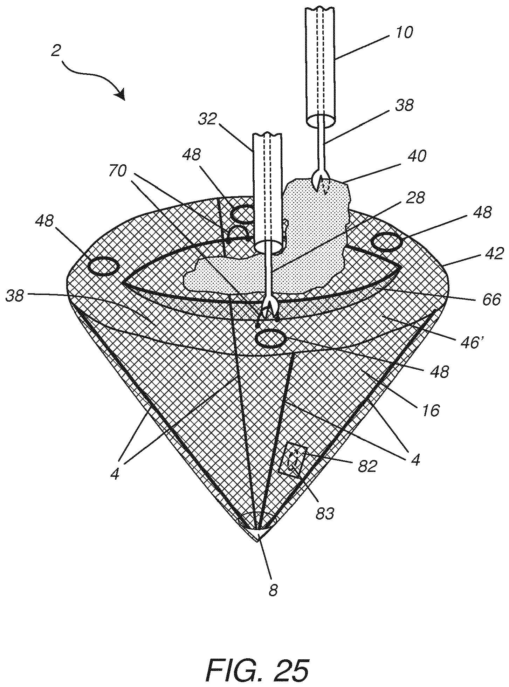

FIG. 25 shows a top perspective view of a specimen **40** being inserted through a closable central opening **68** within a lid **46'.** A grasping instrument **28** is fitted through a trocar sleeve **32** and exposes the central opening **68** (by either grasping the flap **66** or the loop **70,** as shown in FIG. 25), to allow the specimen **40** to be inserted into the receptacle **2** by another instrument **38** inserted through a trocar sleeve **10.** According to further embodiments, the receptacle **2** can include a valve **83** to release air from within to aid with collapsing. The air can be released through the valve **83** as the receptacle **2** is collapsing. According to preferred embodiments, the valve **83** can be a one-way exhaust valve, such as a simple loose covering of pliable material **82** over a hole or a slit in the receptacle. More specifically the sides of the of the pliable material **82** covering can be attached to the receptacle **2** to allow air to be released from the valve **83** such as along 1-3 of its sides, but not sealed along all four sides. If the valve covering is another shape than a quadrilateral, not all of its perimeter will be sealed to the receptacle. The valve **83** can be located on any suitable spot in the receptacle 2 but is preferably located near the distal base **8.**

According to more detailed embodiments, the valve **83** is between 2-10 cms from the distal base, such as at about 4 cm from the distal base. According to these embodiments, as the proximal and middle sections of receptacle **2** are being pulled out of the patient through the sleeve **10,** the air within the receptacle **2** can be released through the valve **83,** such as when the lid **46'** is pressed against the distal end of the trocar sleeve **10.** FIG. 22 provides a close-up view of a preferred valve **83** and covering **82.**

Furthermore, the receptacle **2** can be designed to collapse in height alone, so that there is more room and visibility to place the specimen **40** inside the container prior to morcellation and to better accommodate the bag inside the cavity. Once the specimen **40** is placed in the receptacle the opening can be pulled upward (proximally) lengthening the height in preparation for the morcellation. During power morcellation each cut tissue is limited to a maximum diameter so that they can easily fit through a trocar sleeve of a slightly larger diameter. The procedure is for removal of the cut tissue through the specified sleeve during or after morcellation of the tissue, but prior to the removal of the receptacle **2.** The distal end of the morcellator and the sleeve, from which the tissue will be removed, are within the opening of the receptacle, so that no pieces of cut tissue inadvertently fall outside the receptacle and into the abdominal cavity. Once the specimen has been cut and all the cut pieces removed, the empty bag is collapsed and removed out the same tissue removing sleeve.

Prior to its use, the receptacle can be stored inside a tube that can be used as the trocar sleeve at the time of morcellation. Alternatively, the tube, storing the receptacle, can hold the receptacle in its collapsed position as the receptacle is being inserted through a trocar sleeve previously inserted. According to preferred embodiments, there is a tubular ring around the receptacle, keeping it collapsed. This ring has a larger diameter than the inside of the trocar sleeve. Through its center is a pushrod, the pushrod can be configured to push the collapsed receptacle into the trocar sleeve and then through the tubular ring and trocar sleeve.

Insertion of the receptacle **2** into the patient's body, such as the abdominal cavity, can be accomplished any suitable way, such as by pushing the proximal ends of the collapsed support rods **4** through the hollow channel of the trocar sleeve **10** and out the distal opening **14.** Preferably insertion is by pushing the distal end with base **8** though the proximal end of the sleeve **10** thereby collapsing the rods **4** and receptacle **2.** Removal of the receptacle **2** through the proximal opening **22** of the trocar sleeve 10 can be done using any suitable way. As one non-limiting example, a medical practitioner can inwardly pull on filaments **6** attached to the receptacle **2** and positioned outside of the patient's body. Preferably, removal is by pulling on the drawstring **42** on the proximal end through the sleeve **10,** thereby collapsing the receptacle **2,** so that the receptacle can be pulled out proximal end first. According to further embodiments, the distal cap **8** of the receptacle can be attached to a distal cable **58** that a practitioner can pull on from inside the cavity to aide in the insertion of the receptacle **2.** While the receptacle **2** is configured to be inserted distal end **8** first, according to certain embodiments it can be extracted either distal **8** or proximal end **18** first. For example, after closing the drawstring **42,** the receptacle can be inverted and pulled up by the distal end first without spilling the contents of the receptacle **2.**

For embodiments utilizing the attached lid **46',** certain embodiments entail a user pulling proximally on the central tabs or loops (e.g., **70, 64, 84**) on the lid **46'** through a trocar sleeve (e.g., **10**) and simultaneously pull distally on the distal cable **58.** Alternatively, grasping and pulling the pull cord tab **86** through the pull loop **84** will both tighten the drawstring **42** and simultaneously pull the central section of the lid through the trocar sleeve **10.**

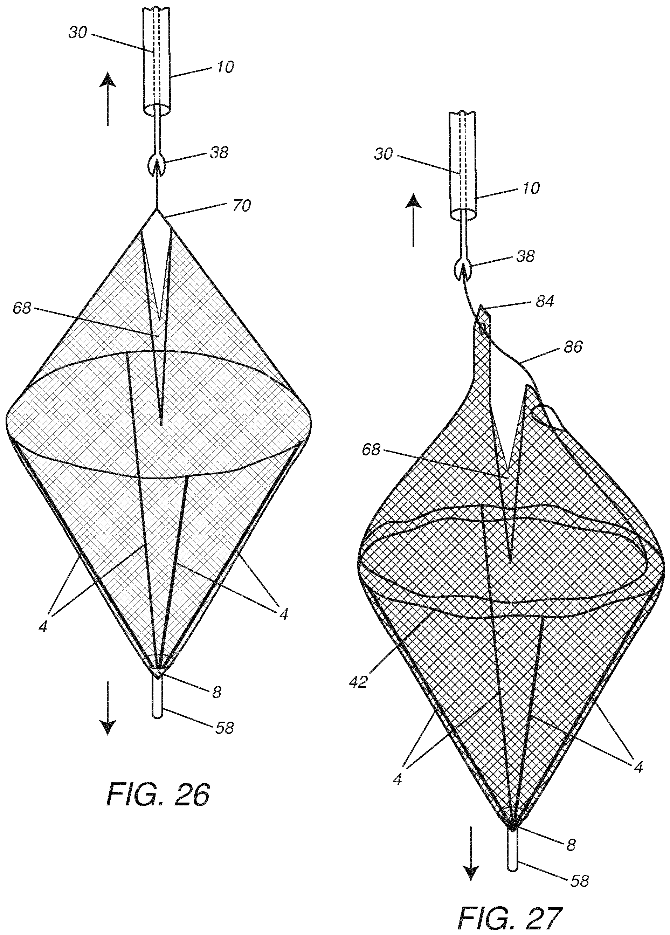

Fig. 26 shows a preferred way of collapsing and removing the receptacle **2** through the trocar sleeve **10.** An instrument **30** extending through the distal opening of a trocar sleeve **10** can grasp a proximal extension member such as a protrusion tab **64,** loop **70** or pull loop **84** and pull proximally towards the trocar sleeve **10.** According to further embodiments, the distal cable **58** can be simultaneously pulled distally with another instrument through another trocar sleeve to assist with collapsing the receptacle **2** such that it will fit through the trocar sleeve **10.**

Preferred methods of morcellating a targeted piece of tissue in a subject include: a) providing a morcellation receptacle as described herein b) creating an incision in the subject near the targeted piece of tissue, wherein the incision has a width of between 14-20 mm and inserting a trocar sleeve into the incision; c) collapsing the receptacle; d) inserting the collapsed receptacle inside of the subject through the trocar sleeve; e) opening the central opening on the permanent lid of the receptacle; f) positioning the targeted tissue into the receptacle through the central opening, then closing said central opening such that cut pieces of the targeted tissue will remain within the receptacle; g) positioning a morcellator into the proximal opening of the receptacle and cutting the target tissue; h) removing the cut targeted tissue from the receptacle through the trocar sleeve; and i) collapsing the receptacle and withdrawing it from inside the subject through the trocar sleeve.

According to preferred embodiments, the targeted specimen **40** has been previously detached from its original points of attachment to the patient, so that it can be positioned into the open receptacle **2.**

The preferred methods of use of the teachings herein are for laparoscopic surgery and are directed for the morcellation receptacle **2** to be collapsed and pushed through a first trocar sleeve **10** that has a diameter of **14** to 20mm and has been placed through the anterior wall into the patient's cavity where it self-expands open, through natural spring tension, into its natural, open position. The targeted specimen **40** that has been previously detached from its points of attachment is positioned into the open receptacle **2** through the proximal opening **18.** The lid **46** can then be collapsed and pushed through the trocar sleeve **10** into the cavity, where it self-expands, and is positioned on top of the proximal opening **18** of the receptacle **2.** According to other embodiments, the lid **46'** or membrane **54** is already attached to the receptacle **2** so they are inserted as one-piece. A rod or other instrument can be used to push the receptacle **4** into and through the trocar sleeve **10.** Afterwards, the specimen **40** is placed into the receptacle **2** through the exposed central opening **68,** the central opening is then closed according to any of the suitable embodiment, such as those described herein, and the specimen **40** is then morcellated within the closed receptacle **2.**

The proximal ends of the receptacle **2** has (3 or 4) filaments **6** attached at their distal ends. The proximal ends of the filaments **6** are grasped through the openings **48** of the lid **46,** and pulled proximally through the anterior wall **12,** on the outside perimeter and adjacent to the trocar sleeves **10, 32, 34,** and **36** that have been previously inserted. Pulling the filaments 6 more proximal pulls the receptacle **2** with specimen **40** inside, and lid **46** on top proximally toward the inside of the anterior wall **12.** The filament **6** sections positioned outside the cavity and proximal to the anterior wall are clamped with clamps **26** so that the receptacle **26** is immobilized or substantially so. The distal end **14** of the first trocar sleeve **10** is positioned through the corresponding hole **48/50** of the lid **46** and positioned so that its distal segment extends into the bag. The second, third and if necessary 4th trocar sleeves **32, 34,** and, **36** are positioned through their corresponding lid openings **48/50** with their distal segments positioned inside the bag. According to further embodiments, the filaments **6** can be released from the clamps **26,** and then the receptacle **2** and attached lid **46'** or membrane **54** are removed as one piece, by pulling, proximally, on the filaments 6 on the center portion of the lid **43'** through the sleeve, and pulling distally on the filament extending from the base of the receptacle, and alternatively, if present, pulling on the drawstring prior to or simultaneously.

As shown in FIG. 15, the proximal ends of the receptacle **2** can also have three filaments **6** attached to the bag at their distal ends. At their proximal ends, the filaments **6** are grasped through the openings of the lid and pulled proximally through the anterior abdominal wall **12** on the outside perimeter and adjacent to the trocar sleeves **32, 34,** and **36** that have been previously inserted. The distal end **14** of the first trocar sleeve **10** is positioned through its corresponding hole.

The electric morcellator **28** is then inserted through second trocar sleeve **32** so that the blade is inside the receptacle **2** and adjacent to the specimen **40** to be cut. For visualization, a laparoscope is inserted through any suitable trocar sleeve **36,** and a first grasper 38 can be inserted through first trocar sleeve **10** and a second grasper **30** can be inserted through a fourth trocar sleeve **34** to stabilize the specimen **40** during morcellation. The morcellated pieces are individually removed from the bag through first trocar sleeve **10,** from which the receptacle **2** itself is inserted and extracted though, which preferably has a larger diameter than the other trocar sleeves. Once the receptacle **2** is empty, the morcellator **28** and graspers **30** and **38** are removed through their respective trocar sleeves **34** and **10.** Additionally the laparoscope is removed from its trocar sleeve **36.** According to preferred embodiments, the laparoscope with corresponding trocar sleeve **36** can remain inside the cavity to maintain visualization as the lid **46** and receptacle **2** are removed. The filaments **6** are released from the clamps **26.** The lid **46** is collapsed and removed through the first trocar sleeve **10.** The drawstring 42 on the proximal end of the bag is pulled into and through first trocar sleeve **10,** collapsing the receptacle **2.** The receptacle **2** is then removed through the first trocar sleeve **10.** Alternatively, if the morcellator has a larger diameter requiring it to slide through trocar **10,** then cut pieces are left in the bag until the desired time to remove them. According to this embodiment, the morcellator can then be removed from the trocar sleeve **10** sleeve and the grasper **38** could be used to remove the cut pieces individually through the trocar sleeve **10.**

The disclosure may be embodied in other specific forms besides and beyond those described herein. The foregoing embodiments are therefore to be considered in all respects illustrative rather than limiting, and the scope of the disclosure is defined and limited only by the appended claims, rather than by the foregoing description.

## Claims

1. A morcellation receptacle system comprising:
a collapsible receptacle (2), having a vertical axis with a lower half section with a distal area, and an upper half section with a proximal end, and a horizontal axis, configured such that when the collapsible receptacle is collapsed on its horizontal axis, it is linearly sized to fit within and through an opening between 14-20 mm in width but not when expanded in a natural open position; said receptacle comprising:
a distal area;
a plurality of at least three support rods (4) positioned in a circumferential and equidistant manner around the vertical axis, or substantially so, and having proximal ends extending proximally and laterally away from the distal area and configured such that the support rods can move towards each other on the horizontal axis when the receptacle is collapsing and move away from each other on the horizontal axis when the receptacle is opening,
a flexible liner (16) that is water resistant, low-friction, tear-resistant, and made of material different from the support rods, having a thickness between .05 - .15 mm, wrapped along the support rods (4) such as to define a bag with a closed end at the distal area;
a plurality of at least two support filaments (6) having distal ends coupled to the upper half section of the receptacle (2) in a circumferential manner and configured such that when proximal ends of the support filaments (6) are pulled away from each other, the receptacle (2) is moved in a proximal direction and is maintained in an open position;
and a cover (46) that is permanently attached to the perimeter of the proximal end of the receptacle (2) that comprises a substantially central opening (68) wherein said opening is configured to be releasably closable such that at least 75% of the opening's area is occluded, and the cover (46) is expandable and collapsible to fit through the 14 to 20 mm opening.

2. The morcellation receptacle system of Claim 1, further comprising complementary sides of the central opening that are configured to releasably bind with each other to close and open the central opening.

3. The morcellation receptacle system of either one of Claim 1 or Claim 2, wherein the cover comprises two zippered tracks (92a, 92b) configured to be opened and closed by a zipper slider (90).

4. The morcellation receptacle system of any preceding claim, wherein the at least two support filaments (6) are spaced equidistantly from each other.

5. The morcellation receptacle system of any preceding claim, wherein the cover (46) comprises a plurality of premade holes (48) configured to allow a trocar sleeve (10, 32, 34, 36) to pass through.

6. The morcellation receptacle system of Claim 5, wherein the at least two support filaments (6) are positioned adjacent to each of the premade holes (48) on the cover.

7. The morcellation receptacle system of any preceding claim, wherein the cover (46) is made of a material that is penetrable by a trocar sleeve (10, 32, 34, 36) through manually applied distal pressure by a practitioner.

8. The morcellation receptacle system of any preceding claim, wherein the cover (46) further comprises a proximal extending member (64, 70, 84, 90) and the distal area comprises a distal extending member (58) configured such that when both are simultaneously pulled proximally and distally, respectively, the receptacle collapses from its expanded configuration;
optionally wherein the proximal extending member (64, 70, 84, 90) is selected from the group consisting of: a proximal protrusion tab (64), a loop (70), zipper slider (90), and a pull loop (84).

9. The morcellation receptacle system of any of Claims 1 to 7, wherein the cover (46) further comprises a centrally located proximal extending member (64, 70, 84, 90) configured such that when pulled proximally through an opening between 14-20 mm in width the receptacle collapses from its expanded configuration;
optionally wherein the proximal extending member (64, 70, 84, 90) is selected from the group consisting of: a proximal protrusion tab (64), a loop (70), zipper slider (90), and a pull loop (84).

10. The morcellation receptacle system of any preceding claim, further comprising a one-way release valve (83) positioned on the flexible liner (16) configured to allow gas to escape from the receptacle (2).

11. The morcellation receptacle system of any preceding claim, wherein the perimeter of the proximal end of the receptacle comprises a drawstring (42) configured to collapse the receptacle (2) from its expanded configuration when pulled away from the perimeter.

12. The morcellation receptacle system of claim 1, wherein the support filaments (6) are coupled to the perimeter of the proximal end of the receptacle (2).

## Patentansprüche

1. Morcellationsbehältersystem, das Folgendes umfasst:
einen faltbaren Behälter (2) mit einer vertikalen Achse mit einem unteren halben Abschnitt mit einem distalen Bereich und einem oberen halben Abschnitt mit einem proximalen Ende und einer horizontalen Achse, der so ausgebildet ist, dass bei Zusammenfalten des faltbaren Behälters entlang seiner horizontalen Achse, dieser linear so dimensioniert ist, um in und durch eine zwischen 14 und 20 mm breite Öffnung zu passen, aber nicht wenn er in einer natürlichen geöffneten Position expandiert ist; wobei der Behälter Folgendes umfasst:
einen distalen Bereich;
eine Vielzahl von zumindest drei Stützstäben (4), die umlaufend und in gleichem Abstand um die vertikale Achse angeordnet sind oder im Wesentlichen derart angeordnet sind und die proximale Enden aufweisen, die sich proximal und lateral von dem distalen Bereich weg erstrecken und so ausgebildet sind, dass die Stützstäbe sich entlang der horizontalen Achse aufeinander zubewegen können, wenn der Behälter zusammengefaltet wird, und sich entlang der horizontalen Achse voneinander weg bewegen können, wenn der Behälter sich öffnet;
eine flexible Auskleidung (16), die wasserbeständig ist, geringe Reibung aufweist, rissfest ist und aus einem anderen Material besteht als die Stützstäbe, eine Dicke zwischen 0,05 und 0,15 mm aufweist, entlang der Stützstäbe (4) so gewickelt ist, dass sie einen Beutel mit einem geschlossenen Ende im distalen Bereich definiert;
eine Vielzahl von zumindest zwei Stützfilamenten (6), die distale Enden aufweisen, die mit dem oberen halben Abschnitt des Behälters (2) umlaufend verbunden sind und so ausgebildet sind, dass, wenn proximale Enden der Stützfilamente (6) voneinander weg gezogen werden, der Behälter (2) in eine proximale Richtung bewegt und in einer geöffneten Position gehalten wird;
und eine Abdeckung (46), die dauerhaft am Umfang des proximalen Endes des Behälters (2) angebracht ist und eine im Wesentlichen zentrale Öffnung (68) umfasst, wobei die Öffnung ausgebildet ist, um lösbar verschließbar zu sein, so dass zumindest 75 % der Fläche der Öffnung verschlossen sind, wobei die Abdeckung (46) expandierbar und faltbar ist, um durch die 14- bis 20-mm-Öffnung zu passen.

2. Morcellationsbehältersystem nach Anspruch 1, das ferner komplementäre Seiten der zentralen Öffnung umfasst, die ausgebildet sind, um sich lösbar miteinander zu verbinden, um die zentrale Öffnung zu verschließen und zu öffnen.

3. Morcellationsbehältersystem nach Anspruch 1 oder 2, wobei die Abdeckung zwei Reißverschlussbahnen (92a, 92b) umfasst, die ausgebildet sind, um durch einen Reißverschlussschieber (90) geöffnet und verschlossen zu werden.

4. Morcellationsbehältersystem nach einem der vorangegangenen Ansprüche, wobei die zumindest zwei Stützfilamente (6) im gleichen Abstand voneinander beabstandet sind.

5. Morcellationsbehältersystem nach einem der vorangegangenen Ansprüche, wobei die Abdeckung (46) eine Vielzahl von vorab hergestellten Löchern (48) umfasst, die ausgebildet sind, um zu ermöglichen, dass eine Trokarhülse (10, 32, 34, 36) durch diese hindurch verläuft.

6. Morcellationsbehältersystem nach Anspruch 5, wobei die zumindest zwei Stützfilamente (6) benachbart in Bezug auf jedes der vorab hergestellten Löcher (48) auf der Abdeckung angeordnet sind.

7. Morcellationsbehältersystem nach einem der vorangegangenen Ansprüche, wobei die Abdeckung (46) aus einem Material besteht, das durch eine Trokarhülse (10, 32, 34, 36) mittels durch einen Arzt manuell ausgeübtem distalem Druck durchstochen werden kann.

8. Morcellationsbehältersystem nach einem der vorangegangenen Ansprüche, wobei die Abdeckung (46) ferner ein sich proximal erstreckendes Element (64, 70, 84, 90) umfasst und der distale Bereich ein sich distal erstreckendes Element (58) umfasst, die so ausgebildet sind, dass sich der Behälter, wenn beide gleichzeitig proximal bzw. distal gezogen werden, aus seiner expandierten Konfiguration zusammenfaltet;
wobei das sich proximal erstreckende Element (64, 70, 84, 90) gegebenenfalls aus der aus einer proximal vorstehenden Lasche (64), einer Schlaufe (70), einem Reißverschlussschieber (90) und einer Zugschlaufe (84) bestehenden Gruppe ausgewählt ist.

9. Morcellationsbehältersystem nach einem der Ansprüche 1 bis 7, wobei die Abdeckung (46) ferner ein zentral angeordnetes, sich proximal erstreckendes Element (64, 70, 84, 90) umfasst, das so ausgebildet ist, dass sich der Behälter, wenn dieses proximal durch eine 14 bis 20 mm breite Öffnung gezogen wird, aus seiner expandierten Konfiguration zusammenfaltet;
wobei das sich proximal erstreckende Element (64, 70, 84, 90) gegebenenfalls aus der aus einer proximal vorstehenden Lasche (64), einer Schlaufe (70), einem Reißverschlussschieber (90) und einer Zugschlaufe (84) bestehenden Gruppe ausgewählt ist.

10. Morcellationsbehältersystem nach einem der vorangegangenen Ansprüche, das ferner ein Einwegablassventil (83) umfasst, das auf der flexiblen Auskleidung (16) angeordnet ist und ausgebildet ist, um zu ermöglichen, das Gas aus dem Behälter (2) austritt.

11. Morcellationsbehältersystem nach einem der vorangegangenen Ansprüche, wobei der Umfang des proximalen Endes des Behälters ein Zugband (42) umfasst, das ausgebildet ist, um den Behälter (2) aus seiner expandierten Konfiguration zusammenzufalten, wenn es von dem Umfang weggezogen wird.

12. Morcellationsbehältersystem nach Anspruch 1, wobei die Stützfilamente (6) mit dem Umfang des proximalen Endes des Behälters (2) verbunden sind.

## Revendications

1. Système de réceptacle de morcellement comprenant :
un réceptacle pouvant être aplati (2), ayant un axe vertical avec une demi-section inférieure avec une zone distale, et une demi-section supérieure avec une extrémité proximale, et un axe horizontal, configuré de telle sorte que lorsque le réceptacle pouvant être aplati est aplati sur son axe horizontal, il est dimensionné de façon linéaire pour s'ajuster dans et à travers une ouverture de 14 à 20 mm de largeur, mais pas lorsqu'il est déployé dans une position naturelle ouverte ; ledit réceptacle comprenant :
une zone distale ;
une pluralité d'au moins trois tiges de support (4) positionnées de manière circonférentielle et équidistante autour de l'axe vertical, ou sensiblement de la sorte, et ayant des extrémités proximales s'étendant de manière proximale et latérale loin de la zone distale et configurées de telle sorte que les tiges de support peuvent se déplacer vers les unes vers les autres sur l'axe horizontal lorsque le récipient est aplati et s'éloignent les unes des autres sur l'axe horizontal lorsque le récipient s'ouvre,
une enveloppe souple (16) qui est résistante à l'eau, à faible frottement, résistante à la déchirure et fabriquée en un matériau différent des tiges de support, ayant une épaisseur comprise entre 0,05 et 0,15 mm, enroulée le long des tiges de support (4) de manière à définir une poche avec une extrémité fermée au niveau de la zone distale ;
une pluralité d'au moins deux filaments de support (6) ayant des extrémités distales couplées à la demi-section supérieure du réceptacle (2) de manière circonférentielle et configurés de telle sorte que lorsque les extrémités proximales des filaments de support (6) sont écartées, le réceptacle (2) est déplacé dans une direction proximale et est maintenu dans une position ouverte ;
et un couvercle (46) qui est fixé de façon permanente au périmètre de l'extrémité proximale du récipient (2) qui comprend une ouverture sensiblement centrale (68) dans laquelle ladite ouverture est configurée pour pouvoir être fermée de manière amovible de sorte qu'au moins 75 % de l'aire d'ouverture la zone sont fermés, et le couvercle (46) peut être déployé et aplati pour s'ajuster à travers l'ouverture de 14 à 20 mm.

2. Système de réceptacle de morcellement selon la revendication 1, comprenant en outre des côtés complémentaires de l'ouverture centrale qui sont configurés pour se lier de manière amovible les uns aux autres pour fermer et ouvrir l'ouverture centrale.

3. Système de réceptacle de morcellement selon l'une des revendications 1 ou 2, dans lequel le couvercle comprend deux glissières de fermeture à glissières (92a, 92b) configurées pour être ouverts et fermés par un curseur de fermeture à glissières (90).

4. Système de réceptacle de morcellement selon l'une quelconque des revendications précédentes, dans lequel les au moins deux filaments de support (6) sont espacés de manière équidistante l'un par rapport à l'autre.

5. Système de réceptacle de morcellement selon l'une quelconque des revendications précédentes, dans lequel le couvercle (46) comprend une pluralité de trous préfabriqués (48) configurés pour laisser passer un manchon de trocart (10, 32, 34, 36) à travers ceux-ci.

6. Système de réceptacle de morcellement selon la revendication 5, dans lequel les au moins deux filaments de support (6) sont positionnés de manière adjacente à chacun des trous préfabriqués (48) sur le couvercle.

7. Système de réceptacle de morcellement selon l'une quelconque des revendications précédentes, dans lequel le couvercle (46) est fait d'un matériau qui peut être pénétré par un manchon de trocart (10, 32, 34, 36) par l'intermédiaire d'une pression distale appliquée manuellement par un praticien.

8. Système de réceptacle de morcellement selon l'une quelconque des revendications précédentes, dans lequel le couvercle (46) comprend en outre un élément d'extension proximal (64, 70, 84, 90) et la zone distale comprend un élément d'extension distal (58) configuré de telle sorte que lorsque les deux sont simultanément tirés de manière proximale et de manière distale, respectivement, le réceptacle s'aplatit à partir de sa configuration déployées ;
facultativement dans lequel l'élément d'extension proximal (64, 70, 84, 90) est sélectionné dans le groupe comprenant : une languette de saillie proximale (64), une boucle (70), un curseur de fermeture à glissières (90) et une boucle de traction (84).

9. Système de réceptacle de morcellement selon l'une quelconque des revendications 1 à 7, dans lequel le couvercle (46) comprend en outre un élément d'extension proximal positionné de manière centrale (64, 70, 84, 90) configuré de telle sorte que lorsqu'il est tiré de manière proximale à travers une ouverture de 14 à 20 mm de largeur, le réceptacle s'aplatit à partir de sa configuration déployée ;
facultativement dans lequel l'élément d'extension proximal (64, 70, 84, 90) est sélectionné dans le groupe comprenant : une languette de saillie proximale (64), une boucle (70), un curseur de fermeture à glissière (90) et une boucle de traction (84).

10. Système de réceptacle de morcellement selon l'une quelconque des revendications précédentes, comprenant en outre une soupape de décharge unidirectionnelle (83) positionnée sur l'enveloppe flexible (16) configurée pour permettre au gaz de s'échapper du réceptacle (2).

11. Système de réceptacle de morcellement selon l'une quelconque des revendications précédentes, dans lequel le périmètre de l'extrémité proximale du réceptacle comprend un cordon de serrage (42) configuré pour aplatir le réceptacle (2) à partir de sa configuration déployée lorsqu'il est tiré à l'écart du périmètre.

12. Système de réceptacle de morcellement selon la revendication 1, dans lequel les filaments de support (6) sont couplés au périmètre de l'extrémité proximale du réceptacle (2).
